# EUROPÄISCHE PATENTANMELDUNG

(11) **EP 2 277 864 A1**
(43) Veröffentlichungstag der Anmeldung: **26.01.2011**
(21) Anmeldenummer: 09008251.2
(22) Anmeldetag: 24.06.2009
(51) Int. Cl.: C07D 213/10

(54) **Syntheseprozess für 3-Methylpyridin**

(71) Anmelder: Lonza Ltd., 4052 Basel (CH)
(72) Erfinder: Die Erfindernennung liegt noch nicht vor

(57) **Zusammenfassung**

Die vorliegende Erfindung betrifft ein Verfahren zur Synthese von 3-Methylpyridin aus Formaldehyd, Paracetaldehyd, Ammoniak und Essigsäure, **dadurch gekennzeichnet, dass** die genannten Verbindungen zur Reaktion gebracht werden und das Verfahren die folgenden Parameter umfasst:
a) eine Reaktionstemperatur von 260-300°C;
b) ein molares Verhältnis von Formaldehyd und Paracetaldehyd von 0.7-1.4 Mol/Mol;
c) eine Ammoniak-Konzentration von 10-20 Gew.-%;
d) eine Essigsäure-Konzentration von 4-20 Gew.-%;
e) eine Paracetaldehyd-Konzentration von 0.4-1.6 Mol/kg; sowie
f) eine Verweilzeit von 10-30 Minuten bei einer kontinuierlichen Reaktionsführung sowie 10 bis 90 Minuten im Falle einer diskontinuierlichen Reaktionsführung; sowie
g) einen Reaktionsdruck von 30 bis 130 bar

## Beschreibung

Die vorliegende Erfindung betrifft ein Verfahren zur Herstellung von 3-Methylpyridin (3-Picolin) aus Formaldehyd, Paracetaldehyd, Ammoniak und Essigsäure.

3-Picolin ist eine farblose, brennbare Flüssigkeit, welche Verwendung als Lösungsmittel, bei der Herstellung von Pharmazeutika und Insektiziden sowie zur Synthese von Nikotinsäure und Nikotinamid findet.

Es sind verschiedene Synthesewege zur Herstellung von 3-Picolin bekannt, welche generell auf einer Additions-/Cyclisierungsreaktion von Aldehyd/Keton Mischungen mit einer Ammoniumverbindung beruhen. Diese Reaktionen können in der Gasphase oder in der flüssigen Phase sowie unter Verwendung eines Katalysators ablaufen.

Das erfindungsgemäße Verfahren baut auf der Veröffentlichung von Grayson, J. und Dinkel, R., "An improved Liquid-Phase Synthesis of Simple Alkylpyridines", Helvetica Chimica Acta, Vol. 67 (1984), S. 2100-2110, auf.

Die Autoren dieser Veröffentlichung beschreiben in Tabelle 2, S. 2108 u.a. die Synthese von 3-Picolin aus Acetaldehyd und Formaldehyd, wobei verschiedene Ammonium-Quellen hinsichtlich des Gehalts an 3-Picolin sowie diverser, unerwünschter Nebenprodukte verglichen werden.

Insbesondere wird gezeigt, dass bei der Verwendung von Ammoniumacetat eine Ausbeute von 44 % erreicht wird, wobei als Hauptnebenprodukt 3-Ethylpyridin in einer Menge von 18 % entsteht.

Es ist die Aufgabe der vorliegenden Erfindung, das Verfahren von Grayson und Dinkel in Bezug auf die Ausbeute an 3-Picolin, Reduktion von 3-Ethylpyridin als Hauptnebenprodukt sowie Raum/Zeit-Ausbeute zu verbessern.

Dieses Problem wird durch das erfindungsgemäße Verfahren zur Synthese von 3-Methylpyridin aus Formaldehyd, Paracetaldehyd, Ammoniak und Essigsäure gelöst, **dadurch gekennzeichnet, dass** die genannten Verbindungen zur Reaktion gebracht werden und das Verfahren die folgenden Parameter umfasst:
a) eine Reaktionstemperatur von 260-300°C;
b) ein molares Verhältnis von Formaldehyd und Paracetaldehyd von 0.7-1.4 Mol/Mol;
c) eine Ammoniak-Konzentration von 10-20 Gew.-%;
d) eine Essigsäure-Konzentration von 4-20 Gew.-%;
e) eine Paracetaldehyd-Konzentration von 0.4-1.6 Mol/kg;
f) eine Verweilzeit von 10-30 Minuten im Falle einer kontinuierlichen Reaktionsführung sowie 10 bis 90 Minuten im Falle einer diskontinuierlichen Reaktionsführung; sowie
g) einen Reaktionsdruck von 30 bis 130 bar

Es kann erfindungsgemäß bevorzugt sein, dass die Reaktion in einem Reaktor stattfindet. Besonders bevorzugt sind hierbei Systeme mit hoher Durchmischungseffizienz wie Rührwerke sowie kontinuierliche Durchflussrührkessel und diskontinuierliche Rührkessel, ganz besonders bevorzugt sind Loop-Reaktoren und Jet-Loop-Reaktoren.

Loop- und Jet-Loop-Reaktoren sind erfindungsgemäß **dadurch gekennzeichnet, dass** die entsprechenden Reaktanden mit Katalyt-Lösung unter kontinuierlicher Fahrweise zur Reaktion gebracht werden. Ein wesentlicher Vorteil von Jet Loop Reaktoren bei der Herstellung von 3-Picolin ist die intensivere und schnellere Flüssigkeitsvermischung bei hoher Umlaufströmung und somit ein erhöhter Wärme- und Stoffübergang als in einem Rührkessel. Bevorzugt wird die erfindungsgemäße Reaktion in einem Strahlzonen-Schlaufenreaktor durchgeführt. Ein weiterer Vorteil ergibt sich bei strahlgetriebenen [Düsen] Schlaufenreaktoren durch eine feinere Dispergierung der zugespeisten Phasen und somit einer größeren spezifischen Phasengrenzfläche.

Des Weiteren kann es erfindungsgemäß bevorzugt sein, dass im Rahmen der Rezirkulierung des Katalysators bei der Reaktion entstehende Nebenprodukte entfernt werden. Hierfür kommen grundsätzlich die im Stand der Technik bekannten Möglichkeiten in Betracht, wie z.B. Extraktion und Rektifikation. Hierbei ist die Destillation erfindungsgemäß besonders bevorzugt.

Erfindungsgemäß können Ammoniak und Formaldehyd sowohl in molekularer Form als auch in Form ihres Additionsproduktes Hexamethylentetramin (Urotropin) zugegeben werden.

Das erfindungsgemäße Verfahren hebt sich vom Stand der Technik gemäß Dinkel et al. u.a. dadurch ab, dass es eine höhere Selektivität im Hinblick auf die Bildung von 3-Methylpyridin mit einer höheren Raum-/Zeit-Ausbeute kombiniert. Damit wurde ein besonderer technischer Fortschritt erzielt, da normalerweise bei der vorliegenden Reaktion eine Erhöhung der Raum-/Zeit-Ausbeute mit einer geringeren Selektivität einhergeht.

Das erfindungsgemäße Verfahren wird durch das folgende Beispiel näher erläutert.

### Beispiel 1

Die Reaktion findet kontinuierlich in einem sehr gut durchgemischten 100 Liter Reaktor statt. Die Zugabe der Katalytlösung (Gemisch Wasser, Ammoniak und Essigsäure) sowie der Edukte (Paracetaldehyd und Formalin) erfolgt mittels Pumpen.

261 kg/h der Katalytlösung (75 Gew.-% Wasser, 15 Gew.-% Ammoniak und 10 Gew.-% Essigsäure) werden mittels Hochdruckpumpe in den Reaktor befördert. Gleichzeitig werden ebenfalls kontinuierlich über Hochdruckpumpen 13 kg/h Paracetaldehyd und 26.8 kg/h Formalinlösung [37.4 Gew.-%] zudosiert. Die Temperatur im Reaktor wird auf 278°C und der Reaktordruck auf 100 bar gehalten. Bei einer Verweilzeit im Reaktor von 20 Minuten erhält man am Reaktoraustritt eine Rohlösung, die 10.02 kg/h 3-Picolin enthält bei einer gleichzeitigen Produktion von 0.37 kg/h 3-Ethylpyridin. Unter diesen Bedingungen wird eine 3-Picolin Ausbeute von 64.6% (basierend auf Formaldehyd) und eine 3-Ethylpyridin Ausbeute von 3.5% (basierend auf Acetaldehyd) erzielt. Alle Pyridinbasen werden per GC gemessen.

## Patentansprüche

1. Verfahren zur Synthese von 3-Methylpyridin aus Formaldehyd, Paracetaldehyd, Ammoniak und Essigsäure, **dadurch gekennzeichnet, dass** die genannten Verbindungen zur Reaktion gebracht werden und das Verfahren die folgenden Parameter umfasst:
a) eine Reaktionstemperatur von 260-300°C;
b) ein molares Verhältnis von Formaldehyd und Paracetaldehyd von 0.7-1.4 Mol/Mol;
c) eine Ammoniak-Konzentration von 10-20 Gew.-%;
d) eine Essigsäure-Konzentration von 4-20 Gew.-%;
e) eine Paracetaldehyd-Konzentration von 0.4-1.6 Mol/kg;
f) eine Verweilzeit von 10-30 Minuten bei einer kontinuierlichen Reaktionsführung sowie 10 bis 90 Minuten im Falle einer diskontinuierlichen Reaktionsführung; sowie
g) einen Reaktionsdruck von 30 bis 130 bar

2. Verfahren gemäß Anspruch 1, **dadurch gekennzeichnet, dass** die Reaktion in einem Reaktor mit hoher Durchmischungseffizienz stattfindet.

3. Verfahren gemäß Anspruch 2, **dadurch gekennzeichnet, dass** es sich um einen kontinuierlichen oder diskontinuierlichen Durchflussrührkessel handelt.

4. Verfahren gemäß Anspruch 2, **dadurch gekennzeichnet, dass** es sich um einen Loop-Reaktor handelt.

5. Verfahren gemäß mindestens einem der Ansprüche 1-4, **dadurch gekennzeichnet, dass** im Rahmen einer Rezirkulierung des Katalysators unerwünschte Nebenprodukte entfernt werden.

6. Verfahren gemäß Anspruch 5, **dadurch gekennzeichnet, dass** besagte Nebenprodukte mittels Rektifikation und Extraktion entfernt werden.

7. Verfahren gemäß mindestens einem der Ansprüche 1-6, wobei die Raum-/Zeit-Ausbeute an 3-Methylpyridin mehr als 50 kg/m³*h, bevorzugt mehr als 80 kg/m³*h und besonders bevorzugt mindestens 100 kg/m³*h beträgt.

8. Verfahren gemäß mindestens einem der Ansprüche 1-7, wobei die Ausbeute an 3-Methylpyridin mindestens 64% (bezogen auf Formaldehyd) und die Ausbeute an Ethylpyridin höchstens 4% (bezogen auf Paracetaldehyd) beträgt.
